# EUROPEAN PATENT APPLICATION

(11) **EP 2 287 154 A1**
(43) Date of publication of application: **23.02.2011**
(21) Application number: 09165400.4
(22) Date of filing: 14.07.2009
(51) Int. Cl.: C07D 215/18, A61K 31/4704, A61P 11/06

(54) **Efficient synthesis for the preparation of montelukast**

(71) Applicant: KRKA, D.D., Novo Mesto, 8501 Novo mesto (SI)
(72) Inventor: Kidemet, Davor, 42000, Varazdin (HR); Benkic, Primoz, 1000, Ljubljana (SI); Kljajic, Alen, 3000, Celje (SI); Stefane, Bogdan, 2317, Oplotnica (SI)
(74) Representative: Wiedemann, Peter

(57) **Abstract**

The present invention describes the improved process for the preparation of montelukast acid and its pharmaceutically acceptable salts and esters. The process is cost effective, environment friendly, and easily scale up to commercial level.

## Description

### Field of the invention

The present invention belongs to the field of organic chemistry. More particularly, the present invention describes the improved process for the preparation of montelukast acid and its pharmaceutical acceptable salts and esters. The process is cost effective, environmentally friendly, and easily scaled up to commercial level.

### Background of the invention

Montelukast sodium has the chemical name 1-[[[[3-[(1E)-2-(7-chloroquinoline-2-yl)ethenyl]phenyl]-3-[2-(1-hydroxy-1-methylethyl)phenyl]propyl]thio]methyl]cyclopropane acetic acid monosodium salt, and it is represented by the formula:

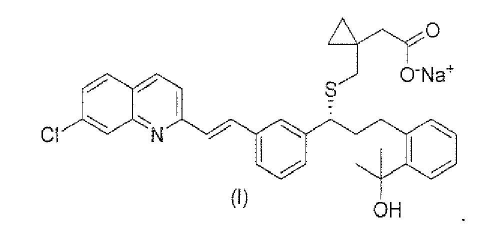

It is a very well known leukotriene receptor antagonist used for the maintenance treatment of asthma and to relieve symptoms of seasonal allergies. Montelukast sodium is marketed in the form of film coated tablets, chewing tablets and granules under the trade name SINGULAIR^{®}.

The substance was first described in EP 480 717 A1. The preparation process of EP 480 717 is as disclosed in the following Scheme 1:

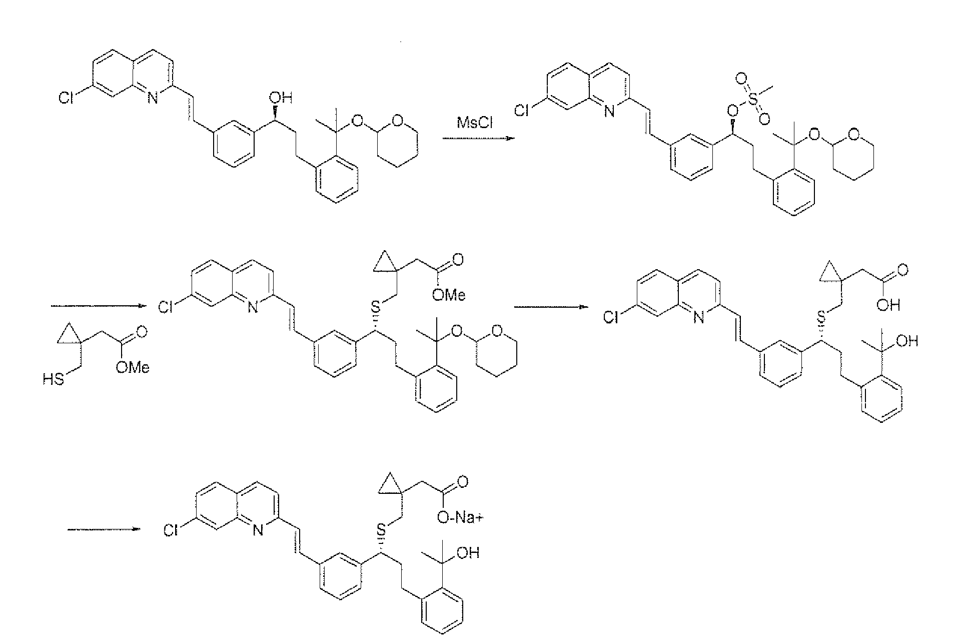

When using the oxazaborolidine complex in earlier reaction steps, a partially over-reduced product is formed in an amount up to 10%.

An improved process is described in EP 737 186 wherein the dilithium salt of 1-(mercaptomethyl)cyclopropaneacetate is reacted with the mesylate derivative. The organic solution of montelukast is transformed to the dicyclohexylammonium salt of montelukast. The drawback of this route of synthesis is the use of n-butyl lithium which is highly reactive and difficult to handle on industrial scale.

Consequently, there still exists a need for an efficient synthesis of montelukast sodium, suitable for large-scale production.

Prior-art applications mainly disclose purification of montelukast acid by formation of amine salts. This is an additional step in production process of pure montelukast acid or its salts that may lead to problems because of carrying over of amine impurities. Therefore, a process of purification via montelukast acid with good yield and good efficiency would be advantageous.

### Summary of the invention

Inventors of the present invention have developed an efficient industrial process for the preparation of pure montelukast acid or its pharmaceutically acceptable salts and esters, wherein montelukast acid is efficiently purified by emulsion crystallization or by supercritical fluid chromatography or by silica gel chromatography or by ion exchange chromatography. By using said purifying processes there is no need for the preparation of any other salts of montelukast acid but only pharmaceutically acceptable montelukast salts, and also there is no need to purify the product using consecutive crystallization processes from different solvents of different polarities to achieve efficient removal of different type of impurities such as for example disclosed in WO 2009/006861.

One aspect of the present invention is thus an efficient process for the production of montelukast acid and its pharmaceutically acceptable salts and esters, particularly sodium salt, comprising the steps of:
a) the synthesis of (R,E)-methyl 2-(3-(acetylthio)-3-(3-(2-(7-chloroquinolin-2-yl)vinyl)phenyl)propyl)benzoate,
b) synthesis of montelukast ester,
c) hydrolysis of montelukast ester to montelukast acid,
d) purification of montelukast acid and
e) optional conversion of montelukast acid into its pharmaceutically acceptable salts or esters.

An additional aspect of the present invention is to provide an efficient process for the purification of montelukast acid that could be performed in one or more of the following ways:
a) by emulsion crystallization,
b) by supercritical fluid chromatography (SFC),
c) silica gel chromatography, and/or
d) ion exchange chromatography.

Another aspect of the present invention is the purification of montelukast acid by emulsion crystallization comprising the following steps:
(i) preparation of an emulsion of montelukast acid,
(ii) preparation of a suspo-emulsion of montelukast acid,
(iii) cooling the suspo-emulsion,
(iv)optionally ageing the suspo-emulsion,
(v) isolation of montelukast acid.

Still another aspect of the present invention is the pharmaceutical composition comprising montelukast acid or its pharmaceutically acceptable salts or esters prepared by the process of the present invention.

### Figures

Figure 1: Two microscope pictures of montelukast acid crystals purified by the method disclosed in example 2.
Figure 2: Particle size distribution of montelukast acid crystals purified by the method disclosed in example 2.

### Detailed description of the invention

The authors of the present invention surprisingly found out that the key step in an improved process for the preparation of montelukast acid and its pharmaceutically acceptable salts and esters according to the present invention is the hydrolysis of montelukast ester without isolation of intermediate products and further purification of montelukast acid according to the present invention that enables to obtain montelukast acid with high purity, cost effective, environmentally friendly, and easily scale up manner.

A preferred variant of the process of the invention is schematically depicted in Scheme 1, wherein

L and L' represent a leaving group, selected form the group consisting of chlorine, bromine or iodine, a C₁-C₈ alkyl sulfonyloxy group or a substituted C₅-C₁₀ aryl sulfonyloxy group; and

R represents a C₁-C₈ alkyl group or a substituted C₅-C₁₀ aryl group.

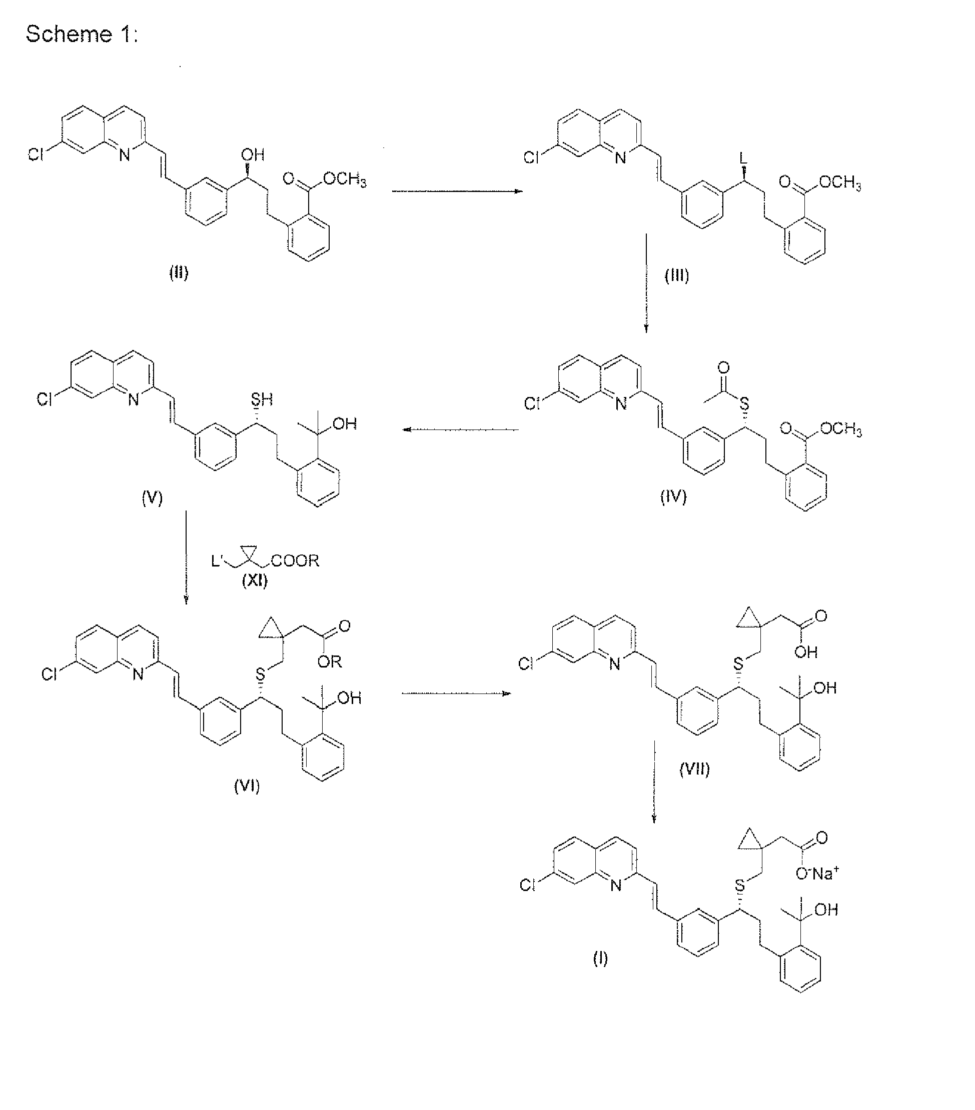

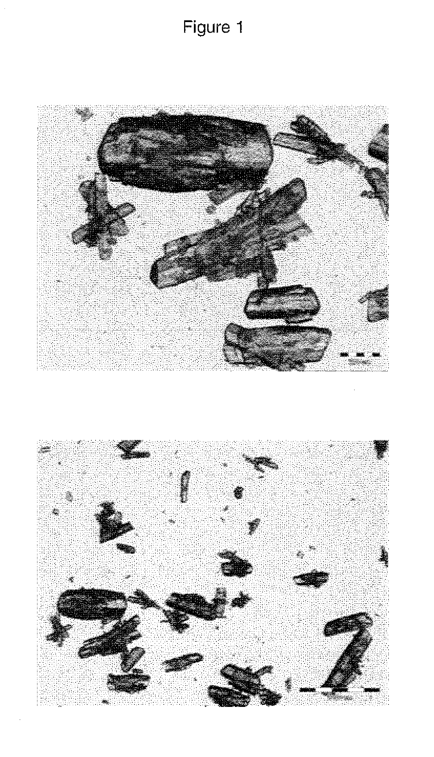

It is also possible in accordance with the present invention to carry out a reaction sequence for the synthesis of montelukast free acid (compound (VII)) or a pharmaceutically acceptable salt or ester thereof (exemplified in Scheme 1 as the sodium salt (I)), which omits one or more of the individual reaction steps depicted in Scheme 1. For instance, it is in accordance with the present invention to use a compound (IV) or (V), which is obtained from other sources or via alternative methods, as a starting material to carry out the subsequent steps shown in Figure 1.

In one aspect, the present invention provides an efficient process for the production of montelukast acid and its pharmaceutically acceptable salts and esters, particularly sodium salt, comprising the steps of:

### a) Synthesis of (R,E)-methyl 2-(3-(acetylthio)-3-(3-(2-(7-chloroquinolin-2-yl)vinyl)phenyl)propyl)benzoate

(S,E)-methyl2-(3-(3-(2-(7-chloroquinolin-2-yl)vinyl)phenyl)-3-hydroxypropyl) benzoate monohydrate (compound of formula (II) is dried by azeotropic distillation. The resulting suspended material is converted to activated compound of formula (III). The intermediate compound of formula (III) is further substituted with potassium thioacetate to afford (R,E)-methyl 2-(3-(acetylthio)-3-(3-(2-(7-chloroquinolin-2-yl)vinyl)phenyl)propyl)benzoate (compound of formula IV). The reaction is performed in one pot without isolation of intermediate compound of formula (III).

### b) Synthesis of montelukast methyl ester

(R,E)-methyl 2-(3-(acetylthio)-3-(3-(2-(7-chloroquinolin-2-yl)vinyl)phenyl)propyl)benzoate (compound of formula IV) is then reacted with methylmagnesium halogenide, such as for example iodide or bromide, to afford (R,E)-2-(2-(3-(3-(2-(7-chloroquinolin-2-yl)vinyl)phenyl)-3-mercaptopropyl)phenyl)propan-2-ol (compound of formula V). Catalyst as for example lanthanum (III) chloride or cesium (III) chloride could be used in the reaction. The formation of compound of formula (V) from the compound of formula (IV) could be performed in any organic solvent wherein the catalyst is soluble, preferably toluene is used.

The intermediate (R,E)-2-(2-(3-(3-(2-(7-chloroquinolin-2-yl)vinyl)phenyl)-3-mercaptopropyl)phenyl)propan-2-ol reacts in the nucleophilic substitution reaction with compound of formula (IX) to afford compound of formula (VI).

The obtained crude compound of formula (VI) is further purified by extractions and by filtration through silica.

### c) Hydrolysis of montelukast ester to montelukast acid

Base catalysed hydrolysis of crude and/or purified montelukast ester (compound of formula (VI)) affords crude montelukast acid (compound of formula VII). The preferred bases to provide montelukast acid of high quality and with lower costs are alkaline hydroxides, such as for example sodium hydroxide.

### d) Purification of montelukast acid

The obtained crude Montelukast acid can be directly used in the next step of the synthesis or is supplementary purified:
a) by crystallization/emulsion crystallization,
b) by supercritical fluid chromatography (SFC),
c) silica gel chromatography,
d) ion exchange chromatography.

Preferably crystallization using ethyl-acetate and water as solvents and Aerosol OT-B as the surfactant for crystallization control is used.
e) Optional conversion of montelukast acid into its pharmaceutically acceptable salts or esters.

Finally, the crude or purified montelukast acid is transformed to its pharmaceutically acceptable salts and esters, particularly sodium salt with ethanolic sodium hydroxide. Precipitation of Montelukast sodium from n-heptane and toluene gives active substance as amorphous Montelukast sodium. The product is finally dried to obtain the final product.

According to one aspect of present invention, the (R,E)-methyl 2-(3-(acetylthio)-3-(3-(2-(7-chloroquinolin-2-yl)vinyl)phenyl)propyl)benzoate (compound of formula IV) can be prepared from compound of formula (II) with chemical name (S,E)-methyl 2-(3-(3-(2-(7-chloroquinolin-2-yl)vinyl)phenyl)-3-hydroxypropyl) benzoate monohydrate by azeotropic distillation of monohydrate compound of formula II with toluene, followed by activating of alcohol with activating group (sometimes also referred to as leaving group) and substitution of the intermediate derivative with potassium thioacetate to afford compound of formula (IV). Activating group may be selected from, but not limited to, chlorine, bromine, iodine or a C₁-C₈ alkyl sulfonyloxy group or a substituted C₆-C₁₀ aryl sulfonyloxy group, preferably methylsulfonyloxy group is used. The reaction can be performed with an alkyl or aryl sulfonyl halide selected from the group consisting of but not limited to, methyl, ethyl, n-butyl, besyl, o-nosyl, p-nosyl or o,p-nosyl sulfonyl halide in an inert solvent in the presence of a base such as for example any organic tertiary non-nucleophilic base such as for example triethylamine, N-ethyldiisopropylamine, or similar bases. The suitable inert solvent may be selected from dichloromethane, tetrahydrofurane, 2-methyltetrahydrofurane, N,N-dimethylformamide and toluene.

The reaction temperature is typically below the boiling temperature of the solvent used, preferably between about -78°C to boiling temperature of the solvent, more preferably between about -20°C to about 0°C. Preferably methanesulfonyl chloride in toluene is used.

Alternatively, the reaction can be performed with a halogen acid or an inorganic acid halide selected from the group consisting of but not limited to HCI, HBr, HI, SOCl₂, PCl₃, POCl₃, PBr₃ in a suitable solvent. The suitable solvents may be selected from dichloromethane, tetrahydrofurane, 2-methyltetrahydrofurane, toluene and N,N-dimethylformamide. The reaction temperature is typically below the boiling temperature of the solvent used, preferably between about -78°C to boiling temperature of the solvent, more preferably between about -10°C to about 35°C.

Intermediate activated alcohol of formula (III) is further transformed to compound of formula (IV) by nucleophilic substitution with potassium thioacetate in a suitable solvent. The solvent can be selected from the group of solvents, consisting of, but not limited to, benzene, toluene, tetrahydrofurane, 2-methyltetrahydrofurane, dioxane, acetonitrile, N,N-dimethylformamide, N,N-dimethylacetamide or any combination of two abovementioned solvents. Preferably, a 1:1 mixture of tetrahydrofurane and toluene is used.

Compound of formula (IV) is then converted to compound of formula (V) with chemical name (R,E)-2-(2-(3-(3-(2-(7-chloroquinolin-2-yl)vinyl)phenyl)-3-mercaptopropyl)phenyl)propan-2-ol. This can be accomplished by reaction with an organometallic reagent, usually in the presence of a lanthanide metal catalyst in an inert solvent. The reaction temperature is below the boiling temperature of the solvent used, preferably between about -78°C to boiling temperature of the solvent, more preferably between about -20°C to about 25°C.

The organometallic reagent can be selected from the group consisting of, but not limited to, methylmagnesium chloride, methylmagnesium bromide, methylmagnesium iodide or methyllithium. Methylmagnesium iodide and methylmagnesium bromide is preferably used. The organometallic reagent can be added in 2 to 10 equivalents according to compound of formula (IV), preferably 5 to 8 equivalents.

The preparation of tertiary alcohols from esters and Grignard reagents is well known, however in certain cases undesired reaction (e.g. enolization, reduction and condensation) compete with formation of the alcohol. The inventors surprisingly found out that lanthanide salts soluble in organic solvents in catalytic quantities minimize competitive reactions such as enolization and reduction (by β-hydride transfer). Therefore, the lanthanide metal catalyst can be selected form the group of lanthanide (III) halogenides. More preferably lanthanide (III) halogenides can be selected from anhydrous lanthanum (III) chloride or cerium (III) chloride, the most preferably solution of lanthanum (III) chloride and lithium chloride in tetrahydrofurane. The lanthanide metal catalyst can be added in 0.1 to 1.5 equivalents according to the compound of formula (IV) the most preferably 0.4 to 0.8 equivalents.

The inert solvent can be selected from a variety of known process solvents. Illustrative of the solvents that can be utilized either singly or in combinations are tetrahydrofurane, 2-methyltetrahydrofurane, diglyme, dioxane, diethyl ether, diisopropyl ether, tert-butyl methyl ether, cyclopentyl methyl ether and toluene, preferably 2-methyltetrahydrofurane and toluene.

According to another aspect of the present invention the nucleophilic substitution of the compound of formula (V) is performed by reaction the compound of formula (V) with compound of formula (XI) in the presence of a base and in a solvent to obtain the compound of formula (VI) - montelukast ester.

The base can be selected from the group consisting of, but not limited to, an alkali hydroxide, an alkaline earth hydroxide, alkali carbonate, alkali alkoxide, alkali hydride, alkyl lithium or lithium hexamethyldisilazide, preferably sodium methoxide is used.

The solvent can be selected from the group consisting of, but not limited to, benzene, toluene, tetrahydrofuran, dioxane, acetonitrile, N,N-dimethylformamide, N,N-dimethylacetamide, ethanol, methanol, propanol, water, 2-methyltetrahydrofuran, diethoxymethane, or N-methylpyrrolidinone.

According to another aspect of the present invention, the hydrolysis of compound of formula (VI) into montelukast acid is performed in the presence of base in a suitable solvent.

The base can be selected from the group consisting of, but not limited to, an alkali hydroxide, an alkaline earth hydroxide, alkaline carbonate or alkaline alkoxide, preferably sodium hydroxide in water is used.

The solvent can be selected from the group consisting of, but not limited to, tetrahydrofuran, dioxane, acetonitrile, N,N-dimethylformamide, N,N-dimethylacetamide, ethanol, methanol, propanol, water, 2-methyltetrahydrofuran, diethoxymethane, or N-methylpyrrolidinone or any mixture of abovementioned solvents. Preferably tetrahydrofurane and methanol mixture is used.

The montelukast free acid prepared according to the process of the present invention can be directly used in the next step of the synthesis or can be supplementary purified.

According to another aspect of the present invention montelukast free acid is purified by the following methods:
a) by crystallization/emulsion crystallization,
b) by supercritical fluid chromatography (SFC),
c) silica gel chromatography,
d) ion exchange chromatography.

Another aspect of the present invention is the purification of montelukast acid by emulsion crystallization comprises the following steps:
(i) preparation of an emulsion of montelukast acid,
(ii) preparation of a suspo-emulsion of montelukast acid,
(iii) cooling the suspo-emulsion,
(iv) optionally ageing the suspo-emulsion,
(v) isolation of montelukast acid.

The emulsion crystallization can be performed in one or more repeated crystallization cycles. Starting montelukast acid can be prepared by the process according to the present invention or by any process known from the prior art.

The term an emulsion of montelukast acid refers to two phase system, comprising a droplet phase and a continuous phase and optionally any other known emulsion phase like micellar structures, W/O/W type emulsion and similar, and being characterized by Free Gibbs energy of droplet formation ΔG more than 0. Montelukast acid can be dissolved in the droplet or in the continuous phase, depending on which basic type of emulsion is present (W/O or O/W). However, the montelukast acid is completely dissolved and there are no montelukast acid particles present at this point.

The droplet phase comprises droplets with an average size between around 0.05 to around 100 µm.

In one embodiment the droplet phase is composed of an organic solvent which is water insoluble and capable of forming an emulsion with water. As organic solvents aromatic hydrocarbons such as toluene, benzene, halogenated benzene and similar, higher ketones (C₄-C₈) such as diethyl ketone, methyl ethyl ketone, methyl isobutyl ketone, methyl propyl ketone and similar, esters such C₃-C₈ esters and similar, higher alcohols (C₄-C₁₀) such as butanol, hexanol, decanol, tert-butanol and similar and any mixtures thereof can be used. The continuous phase is composed of water, which acts as the anti-solvent phase.

In another embodiment the continuous phase is composed of an organic solvent which is water insoluble and capable of forming an emulsion with water. As organic solvents aromatic hydrocarbons such as toluene, benzene, halogenated benzene and similar, higher ketones (C₄-C₈) such as diethyl ketone, methyl ethyl ketone, methyl isobutyl ketone, methyl propyl ketone and similar, esters such C₃-C₈ esters and similar, higher alcohols (C₄-C₁₀) such as butanol, hexanol, decanol, tert-butanol and similar and any mixtures thereof can be used. The droplet phase is composed of water, which acts as the anti-solvent phase.

When ethyl acetate is used as an organic solvent volume ratio (vol/vol) of organic solvent and water phase can be between 0.18 - 20, preferably 0.5 : 10, most preferably 0.7 - 2.0.

In addition, the emulsion of montelukast acid according to the present invention can comprise one or more additives such as surfactants and dispersants. The additives can be cationic, anionic and non-ionic by their nature, preferably sodium dioctyl sulfosuccinate and sodium benzoate can be used. The main purpose of added additives is to stabilize emulsion and to increase selectivity during the emulsion crystallization process towards highly pure montelukast acid. The additives can be present in an emulsion as a mass fraction (w/w%) of 0.01 -40 %, preferably 0.05 - 10 %, most preferably 0.5- 4%.

The emulsion of montelukast acid according to the present invention is prepared by combining the crude montelukast acid prepared by the process defined above with an organic solvent, such as for example aromatic hydrocarbons such as toluene, benzene, halogenated benzene and similar, higher ketones (C₄-C₈) such as diethyl ketone, methyl ethyl ketone, methyl isobutyl ketone, methyl propyl ketone and similar, esters such C₃-C₈ and similar, higher alcohols (C₄-C₁₀) such as butanol, hexanol, decanol, tert-butanol and similar and any mixtures thereof, preferably an acetate ester solvent can be used, most preferably ethyl acetate, and water solution of one or more additives such as surfactants and dispersants for example cationic, anionic and non-ionic surface active agents, preferably sodium dioctyl sulfosuccinate and sodium benzoate at a pre-defined temperature that is above the temperature at which nucleation of montelukast acid can occur and below the boiling point of the solvents used in the preparation. The montelukast acid, organic solvent, water, additives and any other component can be combined in any order and is not limited with examples disclosed in this invention.

The term suspo-emulsion of montelukast acid refers to a three phase system that is composed of the emulsion of montelukast acid according to the present invention and solid particles of montelukast acid dispersed in said emulsion.

The suspo-emulsion of montelukast acid according to the present invention is prepared by seeding the prepared emulsion of montelukast acid with seed crystals of pure montelukast acid (secondary nucleation process) and/or by particles, which are formed during the primary nucleation process of montelukast acid from the supersaturated emulsion of montelukast acid. The temperature of forming the suspo-emulsion can be between 5 and 70 °C below the temperature, at which all montelukast acid is dissolved in the emulsion of montelukast acid.

Seed crystals of pure montelukast acid can be prepared by any known process disclosed in the prior art, such as for example from CN 1420113, CN 1428335, WO03066598, WO 2005/040123, WO2005073194, WO 2005/074935. In addition to seeding or primary nucleation any other method of forced nucleation can be used, such as for example ultrasound and similar methods, to efficiently obtain suspo-emulsion from the supersaturated emulsion of montelukast acid.

For efficient control of selectivity of emulsion crystallization during the cooling of suspo-emulsion an optimized cooling ramp of crystallization media is performed. It was found out that the highly pure montelukast acid is obtained, if the dynamics of cooling of the formed suspo-emulsion is less than 1.5 K/min, preferably less than 1 K/min, more preferably less than 0.5 K/min. Any continuous function of reactor temperature versus time with above defined average cooling rate can be used during the emulsion crystallization process. The final targeted temperature is between 2 and 60 °C below the temperature at which suspo-emulsion was formed. When the final targeted temperature is achieved optionally ageing of the suspo-emulsion is performed until desired yield of crystallized montelukast acid is achieved.

The obtained product, montelukast acid is isolated by any suitable known method from the art and washed with water.

The purification of montelukast acid by emulsion crystallization according to the present invention provides particles of montelukast acid with an average size of the particles above 50µm, preferably above 100µm, most preferably above 200µm. Moreover, the montelukast acid is obtained with high yield (>80%, preferably >85%, most preferably >90%), with a high purity (HPLC purity >98%, preferably >99%, most preferably >99.5%). The particle size was determined by laser light diffraction method using a Malvern Mastersizer MS 2000 with vegetable oil as the dispersion medium.

The term crude Montelukast acid refers to montelukast acid with mass fraction of montelukast acid (w/w% assay) at least 30%, preferably >50%, most preferably >70 % w/w assay, which can be determined by any appropriate analytical method like for example HPLC, NMR, LC-MS, IR according to commonly used procedures.

The obtained particles of montelukast acid possess excellent filterability properties and are substantially free of agglomerates that are advantageous for large-scale manufacturing process. Montelukast acid particles obtained according to the present invention have excellent properties of bulk material like low triboelectric chargeability, low bulk volume, low hygroscopicity and improved stability that are all properties advantageous for storing montelukast acid as an intermediate in the preparation of pharmaceutically acceptable salts or esters thereof or when montelukast acid is directly used in the preparation of the pharmaceutical composition.

The purification of montelukast acid by silicagel chromatography comprises the following steps:
(i) preparation of liquid sample of montelukast acid
(ii) chromatography
(iii) isolation of montelukast acid.

For the purification of the montelukast acid by chromatography different silicagel stationary phases can be used with particles between 5-300 µm, preferably 10-150 µm and most preferably 10-63 µm.

For the purification of the montelukast acid by chromatography different mobile phases can be used. According to the present invention normal phase chromatography was used where organic solvents such as for example heptane, hexane, toluene, chloroform, dichloromethane as lower polarity solvents in mobile mixture can be used and ethanol, methanol, isopropanol, acetone and methyl ethyl ketone as higher polarity solvents. In our experiments combinations such as toluene/acetone and dichloromeihane/methanol were preferably used and combination dichloromethane/methanol with 10% addition of 25% solution of ammonia in water was most preferably used.

The montelukast acid prepared and isolated according to the process by the present invention can be used in the pharmaceutical composition as the active substance together with other pharmaceutically acceptable excipients or it can be further converted without isolation into any known pharmaceutical acceptable salt as for example disclosed in EP 480717 B1, WO 0006585, WO 2006008751, WO 2006043846, WO 2006064269, WO 2007096875, WO 2007107297. Preferably the pharmaceutical acceptable salt is sodium salt prepared by any method known in the art and being in amorphous or crystalline form as disclosed in for example EP 737188 B1, WO 03066098, WO 2004091618, WO 2004108679, WO 2005075427, WO 2005074893, WO 2007005965, WO 2007012075, WO 2007059325, WO 2007116240. Preferably the sodium salt of montelukast is prepared from montelukast acid prepared by the process of the present invention by reacting the pure montelukast acid in a polar protic solvent with a source of sodium ion followed by evaporation of the solvent and triturating of the residue with non-polar solvent to obtain the sodium salt of montelukast. The polar protic solvent may be selected form the group consisting of ethyl acetate, isopropyl acetate, isobutyl acetate, butyl acetate, methanol, acetonitrile, toluene, and the any mixture thereof. Preferably toluene is used. The source of sodium ion may be selected from the group consisting of sodium hydroxide, sodium methoxide, sodium ethoxide, sodium tert-butoxide, preferably sodium hydroxide. The non-polar solvent may be selected from n-hexane, n-heptane, cyclohexane, methyl tert-butyl ether, cyclopentyl methyl ether, diisopropyl ether. Preferably, n-heptane is used.

The compound methyl 2-(1-(bromomethyl)cyclopropyl)acetate used in the step of synthesis of montelukast methyl ester can be prepared by any method known from the prior art, preferably by the method as presented in Scheme 2. 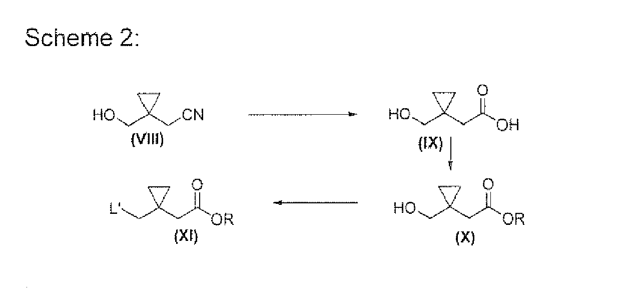 wherein R is as defined above.

The present invention is illustrated by the following Examples without being limited thereto. With the examples we demonstrate the advantage of the process according to the present invention.

### Example 1: Synthesis of Montelukast acid

### Synthesis of (R,E)-methyl 2-(3-(acetylthio)-3-(3-(2-(7-chloroquinolin-2-yl)vinyl)phenyl)propyl)benzoate

(S,E)-methyl 2-(3-(3-(2-(7-chloroquinolin-2-yl)vinyl)phenyl)-3-hydroxypropyl benzoate (50 g, 0.109 mol) and toluene (1 L) are charged into reactor. Half of the amount of toluene is distilled off under reduced pressure to azeotropically dry substance. The solution is cooled to about -10°C and 4-dimethylaminopyridine (1 g, 8.19 mmol) and triethylamine (28 mL, 0.201 mol) are added followed by the addition of methanesulfonyl chloride (11.7 mL, 0.109 mol) over 1-2 hrs while the internal temperature is maintained at about -10°C. When addition has been completed the solution is stirred at -10°C for 3 hrs then quenched into cold saturated sodium hydrogencarbonate aqueous solution (400 mL). The organic layer is washed twice with cold saturated aqueous sodium hydrogencarbonate solution (400 mL) and than dried with anhydrous sodium carbonate. The solution is filtered off into reactor charged with potassium thioacetate (15.57 g, 0.136 mol) in tetrahydrofuran (500 mL). The reaction mixture is stirred at 45-50°C for 24 hrs and than at 20-30°C for another 24 hrs. The reaction mixture is poured into saturated aqueous solution of sodium chloride (400 mL). Layers are separated and aqueous layer extracted twice with toluene (300 mL). Combined organic layers are washed with saturated aqueous sodium chloride solution (300 mL). The solvent is evaporated to about ¼ of the starting volume.

### Synthesis of (R,E)-methyl 2-(1-((1-(3-(2-(7-chloroquinolin-2-yl)vinyl)phenyl)-3-(2-(2-hydroxypropan-2-yl)phenyl)propylthio)methyl)cyclopropyl)acetate

(R,E)-Methyl 2-(3-(acetylthio)-3-(3-(2-(7-chloroquinolin-2-yl)vinyl)phenyl)propyl) benzoate (52.64 g, 0.102 mol) is dissolved in toluene (500 mL), degassed, and 15% solution of lanthanum chloride and lithium chloride in tetrahydrofuran (68 mL, 42.2 mmol) is added, and the suspension is stirred at room temperature for 1 hr. Thereafter, the reaction mixture is cooled to -10 0°C and 3 M solution of methylmagnesium iodide in diethyl ether (204 mL, 0.612 mol) is added dropwise during 1-2 hr while maintaining the temperature below -10°C. Temperature is gradually raised to 10 to 25°C and maintained for several hours. Reaction is poured into cold sat. NN₄Cl (600), and randalite (100 g) is added. Suspension is filtered off, layers separated and aqueous layer extracted with toluene. Combined organic layers are washed with saturated NaHCO₃ (400 mL), and solvent evaporated (temperature bellow 40°C). Residue is dissolved in dimethylformamide (400 mL), cooled to -10°C, and solution of NaOMe (5.97 g, 0.110 mol) in methanol (50 mL) is added dropwise during 1 hr, than solution of methyl 2-[1-(bromomethyl)cyclopropyl]acetate (26.4 g, 0.127 mol) in dimethylformamide (50 mL), and mixture stirred at room temperature for 5 to 24 hrs. Reaction is poured into saturated NaCl (500 mL), and extracted twice with ethyl acetate (400 mL). Combined organic layers are washed with saturated NaHCO₃ (500 mL), dried (Na₂SO₄) and solvent evaporated to afford (R,E)-methyl 2-(1-((1-(3-(2-(7-chloroquinolin-2-yl)vinyl)phenyl)-3-(2-(2-hydroxypropan-2-yl)phenyl)propyl-thio)methyl)cyclopropyl)acetate. Crude product may be purified by column chromatography on silica with hexane-ethyl acetate as an eluent.

### Synthesis of Montelukast acid

(R,E)-methyl 2-(1-((1-(3-(2-(7-chloroquinolin-2-yl)vinyl)phenyl)-3-(2-(2-hydroxypropan-2-yl)phenyl)propylthio)methyl)cyclopropyl)acetate (1.525 kg, 2.54 mol) is charged into reactor, dissolved in tetrahydrofuran (8 kg) and methanol (10 kg). The solution is cooled to 5 to 10°C, and 1M NaOH solution (5.2 kg) is added dropwise maintaining the temperature below 10°C (approximately 1 hr). Thereafter, the reaction mixture is stirred at room temperature for about 24 hrs. Reaction is poured into mixture of saturated NaCl solution (22 kg) and ethyl-acetate (23 kg). Layers are separated and organic layer is washed with 0.5 M aqueous solution of tartaric acid (23 kg) and several times with water (18 kg). The solvent is concentrated *in vacuo,* and residue is dissolved in ethyl acetate followed by slow addition of hexane at room temperature. Thereafter, crystal product is filtered off, washed, and dried under reduced pressure at 40°C to afford montelukast acid.

### Example 2: Purification of montelukast acid by emulsion crystallization

### First crystallization:

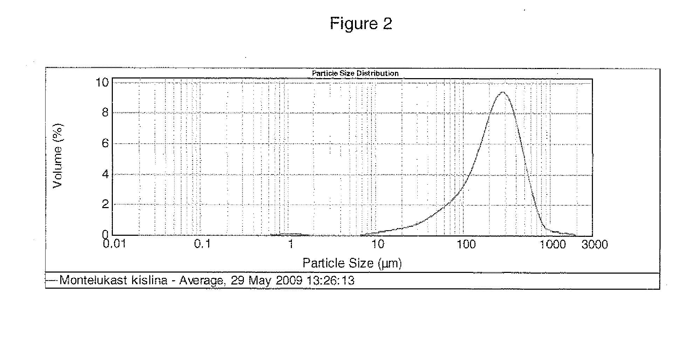

3.15 kg of crude montelukast acid (purity 93.5%, content of impurity 4 ((R,E)-methyl 2-(1-((1-(3-(2-(7-chloroquinolin-2-yl)vinyl)phenyl)-3-(2-(2-hydroxypropan-2 yl)phenyl)propylthio)methyl)cyclopropyl)acetate) was 0.15 %) is completely dissolved in 9 L of ethyl acetate at reflux conditions. 12 L of water and 0.35 kg of DSS (85% w/w dioctyl sodium sulfosuccinate, 15% w/w sodium benzoate) were added. Furthermore, the emulsion was cooled to 45 °C (RT) with cooling ramp 0.5 K/min and after that the emulsion was slowly cooled with the cooling ramp 0.10 K/min to 15 °C (TRCT). The seeds of pure montelukast acid were added at 30 °C (RT), during the slow cooling. Formed suspo-emulsion was homogenized at this temperature for the next 3 hours for montelukast acid to fully crystallize. The formed product is isolated with filtration and washed with water. The microscope pictures of formed product are presented in Figure 1 and particle size distribution in fig 2.

81% cycle yield with the (R,E)-methyl 2-(1-((1-(3-(2-(7-chloroquinolin-2-yl)vinyl)phenyl)-3-(2-(2-hydroxypropan-2-yl)phenyl)propylthio)methyl)cyclopropyl) acetate impurity content of 0,02% and 97.9% pure montelukast content was obtained.

### Second crystallization:

The steps of the first crystallization were repeated. 87% cycle yield with 99.3% pure montelukast acid was obtained with the (R,E)-methyl 2-(1-((1-(3-(2-(7-chloroquinolin-2-yl)vinyl)phenyl)-3-(2-(2-hydroxypropan-2-yl)phenyl)propyllthio)methyl)cyclopropyl) acetate impurity content < 0.01%

**Table 1: HPLC analysis of crystallized materials after the first and second crystallization cycle.**

| | ***Impurity content (%)*** | ***Purity of crystallized material** (%)* | ***Microscopic pictures of formed particles*** |
|---|---|---|---|
| *First cycle* | 0.02 | 97.2 | Figure 1 |
| Second cycle | <0.01 | 99.3 | / |

### Example 3: Purification of montelukast acid by emulsion crystallization via primary nucleation of montelukast acid

31.5 g of crude montelukast acid (purity 93.5%) is completely dissolved in 90 mL of ethyl acetate at reflux conditions. 120 mL of water and 3.5 g of DSS (85% w/w dioctyl sodium sulfosuccinate, 15% w/w sodium benzoate) were added. Under protection of nitrogen and protection from light, the emulsion was cooled to 50 °C (RT) with cooling ramp 0.5 K/min and after that the emulsion was slowly cooled with the cooling ramp 0.05 K/min to 10 °C (TRCT). Formed suspo-emulsion was homogenized at this temperature for the next 10 hours for montelukast acid to fully crystallize. The formed product is isolated with filtration and washed with water. yield: 88%

The crystallization was repeated and material with purity > 99.5% was obtained.

### Example 4: Purification of Montelukast acid by supercritical fluid chromatography

Supercritical fluid chromatography was performed in the following way:
The sample with chromatographic purity of about 80% was purified in one step to about 99.7% chromatographic purity, all individual impurities were below 0.10%.
Column: Princeton SFC 2-Ethylpiridine
Mobile phase A: CO2
Mobile phase B: Methanol
Gradient:

| Time | Mobile phase A | Mobile phase B |
|---|---|---|
| 0 | 83 | 17 |
| 30 | 83 | 17 |
| 40 | 50 | 50 |

Flow: 10ml/min
Tcol: 40 °C
Post run: 3min
Back pressure: 200 bar
Wavelength: 225 nm
Injection volume: 100 µl (concentration: 320 mg/ml)

### Example 5: Purification of montelukast acid by silicagel chromatography

### Example 5.1

Chromatographic column with dimensions 250x21,2 mm and with stationary phase silica 60 (40-63 µm particles) was used. The following mobile phases were used: dicloromethane/methanol, toluene/acetone and dichloromethane/methanol (with 10% addition of 25% solution of ammonia in water).

The best result was obtained by using dichloromethane/methanol (with 10% addition of 25% solution of ammonia in water) mobile phase. The following gradient on HPLC preparative system was used:

| Time (min) | Mobile phase A (vol. %) | Mobile phase B (vol. %) |
|---|---|---|
| 0-6 | 95 | 5 |
| 6,01-30 | 90 | 10 |
| 30,01-60 | 40% | 60 |

Flow=32 ml/min; λ=280nm

Optimum load on the column in grams: sample: stationary phase=1:150.

With these chromatographic conditions the sample with chromatographic purity of about 80% was purified in one step to:
Montelukast acid: more than 99,0% and all individual impurities below 0,10%.

### Example 5.2

Chromatographic column with dimensions 250x16 mm and with stationary phase Nucleosil 100-10 (Macherey-Nagel with 10 µm particles) was used. The following mobile phases were used: dicloromethane/methanol, toluene/acetone and dichloromethane/methanol (with 10% addition of 25% solution of ammonia in water).

The best result was obtained by using dichloromethane/methanol (with 10% addition of 25% solution of ammonia in water) mobile phase. The following gradient on HPLC preparative system was used:

| Time (min) | Mobile phase A (vol. %) | Mobile phase B (vol, %) |
|---|---|---|
| 0-6 | 95 | 5 |
| 6,01-30 | 90 | 10 |
| 30,01-60 | 40% | 60 |

λ=280nm Flow=18ml/min; λ=280nm

Optimum load on the column in grams: sample: stationary phase =1:120.

With these chromatographic conditions the sample with chromatographic purity of about 80% was purified in one step to:
Montelukast acid: more than 99,0% and all individual impurities below 0,10%.

### Example 6: Synthesis of montelukast sodium

The reactor is charged with toluene (8.8 kg) and montelukast acid (0.6 kg, 1.024 mol) and the reactor is cooled to 0-5°C. The ethanolic solution of sodium hydroxide (47 g of NaOH in 2.2 L of ethanol) is added dropwise to the solution of montelukast acid in toluene during 30 min, maintaining the temperature of solution below 5°C. Thereafter, the reaction mixture is stirred at 20-30°C for 2 hrs, than activated carbon is added and solution stirred at the same temperature for 2 hr. The reaction mixture is filtrated and activated charcoal is washed with the mixture of ethanol and toluene. Filtrate is concentrated to about ¼ of the volume. To the crystallization reactor heptane (10 kg) is added and the solution of Montelukast sodium in toluene is added dropwise for 2 hrs (temperature is 15-25°C), and after the addition, the reaction mixture is stirred for 2 hrs at the same temperature. The solution is transferred to the centrifuge, filtrated and crystalline product is washed with heptane. The material is transferred to a drier, and dried.

The final product is amorphous montelukast sodium.

## Claims

1. Process for preparing montelukast free acid or pharmaceutically acceptable salts thereof, wherein the process comprises the step of providing montelukast free acid, subjecting said montelukast free acid to purification treatment by means of one or more of the following methods:
a) by emulsion crystallization,
b) by supercritical fluid chromatography (SFC),
c) silica gel chromatography, and/or
d) ion exchange chromatography.

2. Process according to claim 1, wherein the purification method is (a) emulsion crystallization, and wherein the purification method comprises the following steps:
(i) preparation of an emulsion of montelukast acid,
(ii) preparation of a suspo-emulsion of montelukast acid,
(iii) cooling the suspo-emulsion,
(iv) optionally ageing the suspo-emulsion,
(v) isolation of montelukast acid,
or the purification method is (c) silicagel chromatography, which comprises the following steps:
(i') preparation of a liquid sample of montelukast free acid,
(ii') chromatography,
(iii') isolation of montelukast acid.

3. Process according to claim 2, wherein the emulsion of step (i) comprises ethyl acetate and water such that ethyl acetate and water are used in a volume ratio (vol/vol) of between 0.18 - 20, preferably 0.5 : 10, most preferably 0.7 - 2.0.

4. Process according to any one of claims 1 to 3, wherein the purification treatment is repeated one or more cycles until the resulting montelukast free acid has a purity of at least 97%, preferably at least 99%, more preferably at least 99.5%.

5. Process according to any one of claims 1 to 4, wherein the montelukast free acid subjected to the purification treatment is obtained by effecting base catalyzed hydrolysis on the montelukast ester (VI) to yield montelukast free acid (VII) : 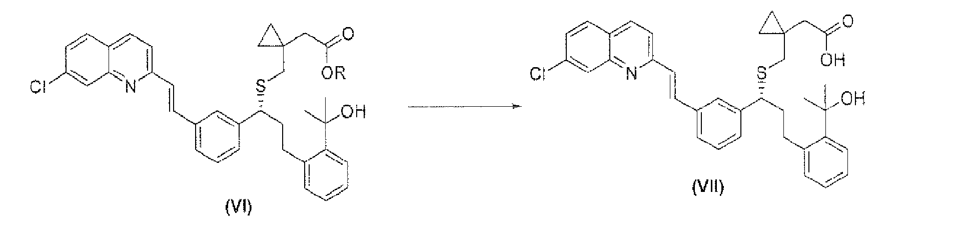 wherein in general formula (VI) R represents a C₁-C₈ alkyl group or a substituted C₅-C₁₀ aryl group, and wherein the hydrolysis is preferably effected using an agent selected from alkali hydroxide, an alkaline earth hydroxide, alkaline carbonate and alkaline alkoxide.

6. Process according to claim 5, wherein the montelukast ester (VI) is obtained by subjecting a compound of formula (V) to nucleophilic substitution reaction with a compound of formula (IX) to afford the montelukast ester of formula (VI): 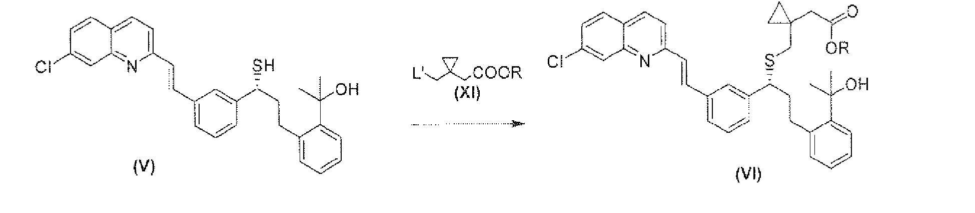 wherein R represents a C₁-C₈ alkyl group or a substituted C₅-C₁₀ aryl group and wherein L' represents a leaving group, selected form the group consisting of chlorine, bromine or iodine, a C₁-C₈ alkyl sulfonyloxy group or a substituted C₅-C₁₀ aryl sulfonyloxy group.

7. Process according to claim 6, wherein the compound of formula (V) is obtained by reacting a compound of general formula (IV) with an organometallic reagent such as methylmagnesium halogenide, preferably in the presence of a catalyst, to afford the compound of formula (V):

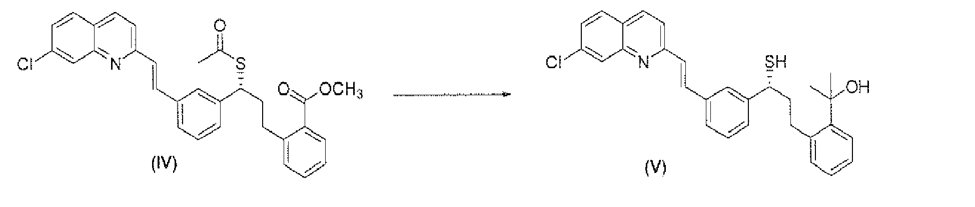

8. Process according to claim 7, wherein the conversion of compound (IV) into compound (V) is effected in the presence of a lanthanide salt catalyst.

9. Process according to claim 7 or 8, wherein the compound of formula (IV) is obtained by reacting a compound of formula (III) with metal thioacetate, preferably potassium thioacetate, to afford the compound of formula (IV): 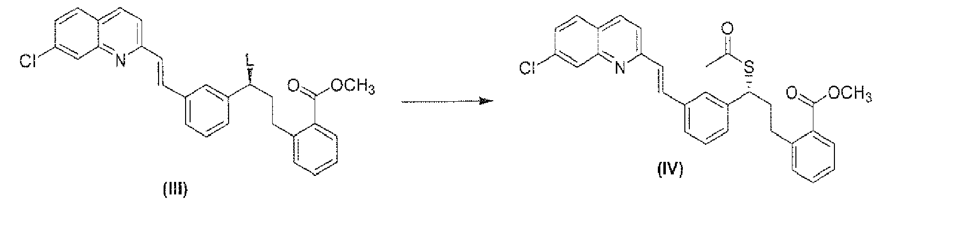 wherein L represents a leaving group, selected form the group consisting of chlorine, bromine or iodine, a C₁-C₈ alkyl sulfonyloxy group or a substituted C₅-C₁₀ aryl sulfonyloxy group.

10. Process according to claim 9, wherein the compound of formula (III) is obtained by drying a compound of formula (II) by azeotropic distillation, followed by conversion of the resulting suspended material into the compound of formula (III): 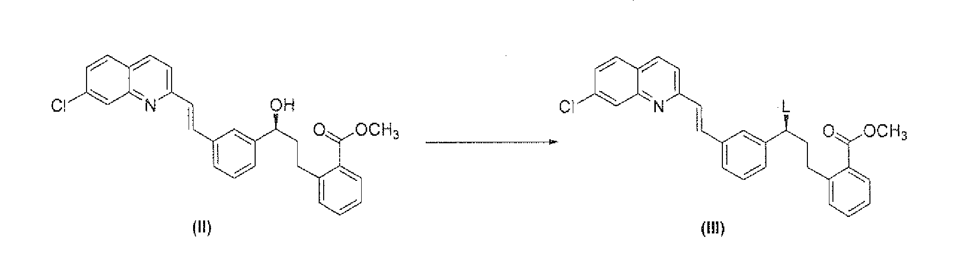 wherein L represents a leaving group, selected form the group consisting of chlorine, bromine or iodine, a C₁-C₈ alkyl sulfonyloxy group or a substituted C₅-C₁₀ aryl sulfonyloxy group.

11. Process according to claim 10, wherein the steps of converting compound (II) into compound (III), followed by conversion of compound (III) into compound (IV) are carried out in a one-pot synthesis without isolation of the intermediate product, compound (III).

12. Process according to any one of claims 1 to 11, which furthermore comprises a step of converting the purified montelukast free acid into its pharmaceutically acceptable salts or esters.

13. Montelukast free acid or a pharmaceutically acceptable salt or ester thereof, which is obtained according to a process as specified in one or more of the claims 1 to 12.

14. Pharmaceutical composition comprising montelukast free acid or a pharmaceutically acceptable salt or ester thereof according to claim 13.

15. Montelukast free acid or a pharmaceutically acceptable salt or ester thereof according to claim 13 or a pharmaceutical composition according to claim 14 for use in the maintenance treatment of asthma and/or to relieve symptoms of seasonal allergies.
